# EUROPEAN PATENT SPECIFICATION

(11) **EP 0 565 789 B1**
(45) Date of publication and mention of the grant of the patent: **16.09.1998**
(21) Application number: 92303405.2
(22) Date of filing: 15.04.1992
(51) Int. Cl.: C07C 17/25, C07C 21/06, B01J 29/06, B01J 29/40

(54) **Production of vinyl chloride by catalytic dehydrohalogenation**
Herstellung von Vinylchlorid durch katalytische Dehydrohalogenierung
Fabrication de chlorure de vinyle par déshydrohalogénation catalytique

(43) Date of publication of application: 20.10.1993
(73) Proprietor: EVC TECHNOLOGY AG, 6300 Zug (CH)
(72) Inventor: Carmello, Diego, I-30175 Mestre-Venezia (IT); Memo, Luisa, I-31022 Preganziol Treviso (IT)
(74) Representative: Votier, Sidney David

(56) References cited:
- EP-A- 2 021
- EP-A- 109 059
- GB-A- 1 152 021
- US-A- 2 803 679
- US-A- 3 702 886
- US-A- 3 856 659
- US-A- 4 384 159
- PATENT ABSTRACTS OF JAPAN vol. 10, no. 128 (C-345)(2185) 13 May 1986

## Description

The present invention relates to a method for the production of vinyl chloride monomer (VCM) from 1,2-dichloroethane. In particular, the invention concerns a process for the catalytic cracking of 1,2-dichloroethane in the presence of a modified zeolite.

Vinyl chloride can be produced from 1,2-dichloroethane (commercially called ethylene dichloride or EDC) by means of pyrolysis by splitting off a molecule of hydrogen chloride. The pyrolysis occurs as a high temperature gas phase free-radical chain reaction. In the standard pyrolysis procedure, a temperature in the range of about 500°C to about 600°C and a pressure of about 500 to 600 psig is used. The vinyl chloride is obtained in the form of vapour and then condensed.

A serious disadvantage of the standard pyrolysis or thermal process is the need of a large amount of expensive energy to heat the EDC to a temperature sufficient to accomplish the cracking. Furthermore, the rapid deposition of solid by-products (coke) on the tubes of the reactor in which cracking takes place limits the process to relatively short operating periods before cleaning of the tubes is required. Furthermore, in these high temperature processes, undesirable side reactions frequently occur which lower the percentage conversion of EDC and result in the formation of undesirable by-products.

To reduce the problems observed in the standard pyrolytic cracking process various catalytic processes have been proposed. The aim of many such catalytic processes is to allow the cracking reaction to proceed at a lower temperature, thus necessitating a lower input of thermal energy.

The catalytic properties of zeolites have long been recognised. Zeolites, or molecular sieves, are crystalline structures composed essentially of [SiO₄]⁴⁻ and [AlO₄]⁵⁻tetrahedral subunits. Arrangement of these units into crystalline formations leads to a structure having a large number of internal channels and passages, which are important in both the sieving and the catalytic properties of the zeolite. The ratio of Si to Al units is variable. Certain synthetic zeolites, such as ZSM-5 and silicalite, may have an extremely high Si:Al ratio.

Cations are present in zeolites as counterions, to neutralise the negatively-charged Si and Al units. Hydrogen and sodium are common counterions, for example as in H-ZSM-5. Zeolites undergo cation exchange very readily, wherein the native counterion is replaced by a foreign ion. This property has been exploited in the design of catalytic zeolites.

However, in a number of catalytic cracking processes where zeolites have been used to catalyse the dehydrohalogenation of EDC to VCM, the yield of VCM is not optimal. In particular, the percentage of EDC which is cracked, referred to as percentage conversion, is frequently low, while the contact time required between the EDC and the catalyst is needlessly high. Furthermore the selectivity of the cracking reaction to yield VCM rather than other, undesirable products is poor.

The prior art describes a number of catalytic processes in which zeolites and modified zeolites are employed as the catalyst. For example in European Patent No. 002,021 a catalyst system comprising a zeolite which has been treated or reacted with a volatile Lewis acid is disclosed for the dehydrochlorination of EDC. Suitable catalysts include Faujasite Y zeolite reacted with TiCl₄. This process resulted in only limited conversion of EDC, thereby requiring long contact times. European Patent No. 162,457 discloses the use of a catalyst system comprising a rare earth metal chloride plus an alkaline metal chloride deposited on a zeolite in the presence of oxygen for the dehydrochlorination process. This process uses very long contact times and obtains only low conversions of EDC and low yields of VCM.

In general, although the prior art processes may be commercially feasible, they still obtain limited conversion of EDC and have low selectivity for VCM production. Furthermore, extended contact times are necessary, which lead to long reaction times. It would be desirable to provide a catalytic system that allows the dehydrochlorination reaction to be carried out with a high conversion of EDC and reduced contact times, without the formation of substantial quantities of by-products and at a relatively low reaction temperature, thereby resulting in reduced energy consumption.

United States Patent No. 4,384,159 teaches the usefulness of zeolites ZSM-5 and S-115 (a silicalite produced by Union Carbide) in dehydrochlorination reactions. Although the zeolites disclosed therein enable shorter contact times than the process of EP 002,021, they provide only limited conversion of EDC. It has now unexpectedly been found that improved conversion and selectivity of the cracking reaction of 1,2-dichloroethane to vinyl chloride can be obtained by controlling the surface acidity and activity of such zeolites by the introduction of Zn and/or Mg ions.

According to a first aspect of the invention, therefore, there is provided a process for the catalytic dehydrochlorination of ethylene dichloride (EDC) to yield vinyl chloride (VCM) comprising:
(i) a cracking step wherein EDC is brought into contact with a catalyst which is a zeolite having a Si:Al ratio of 10 or more and containing an alkaline earth metal ion or a zinc ion; and
(ii) a collecting step in which VCM is obtained in the reaction products.

It will be understood that the zeolite catalyst used in the invention can contain an alkaline earth metal ion or a zinc ion or both in any desired ratio.

It has been found that zeolites having a Si:Al ratio below 10 give poor selectivity to VCM when used for catalytic dehydrochlorination. Preferably, the Si:Al ratio of the zeolite will be between 10 and 1000. An Si:Al ratio above 1000 has been found to have an adverse effect on the level of conversion of the EDC. Advantageously, the Si:Al ratio of the zeolite is between 10 and 800 and preferably between 20 and 200.

Using the modified zeolites of the invention, the dehydrochlorination process may be carried out at lower temperatures, with high selectivity to VCM and longer catalyst life.

Preferably, the zeolite of the invention is ZSM-5 or S-115. Any ZSM-5 zeolite may be used, but the use of H-ZSM-5 in which the native counterion is hydrogen is preferred. Preferably, the alkaline earth metal ion is Mg.

The zeolites which are modified in the present invention are well-known in the art. ZSM-5 has been described in U.S. Patent No. 3,702,886; silicalite (S-115) has been described in U.S. Patent No. 4,061,724. ZSM-5 and silicalite structure have been described by D.H. Olson *et al*. in J. of Catalysis 61, pp. 390-396 (1980).

The synthetic zeolites of the invention are either exchanged or impregnated with zinc or magnesium or their combination in ionic form. The metal content can be varied but optimum results are obtained by using a 10-30% H⁺/M²⁺ exchange ratio that is a 0.2-0.6% w/w content for Mg and a 0.7-2.1% w/w content for Zn. Catalysts have been prepared using methods well-known in the art. Some of these methods are described by D.E.W. Vaughan in Chem. Eng. Progress, February 1988.

Because of the ionic character of the catalytic dehydrochlorination reaction, as opposed to the free-radical mechanism of thermal cracking, in this invention it is possible to use a less pure EDC raw material without any loss of productivity.

The cracking step is performed either in the presence of pure EDC or in an inert gas atmosphere, for example an atmosphere of nitrogen gas.

The temperature in the reactor is preferably maintained in the range of about 200°C to about 400°C, but optimum results are obtained when operating in the range 250°-350°C.

The reactor preferably operates at atmospheric pressure, but a pressure anywhere up to 2026.5 kPa (20 atmospheres) is satisfactory. The contact time of EDC with the catalyst can be varied by changing the space velocity of the gaseous feed in such a way as to obtain the optimum contact time for any particular type of reactor; for example, optimum results may be obtained when operating in the range 0.1 to 5.0 seconds. Preferably, the contact time is between 0.6 and 3.6 seconds. High contact times lead to an increase in by-product formation, while too low contact times give poor EDC conversion.

The invention is now described by way of the following examples.

### COMPARATIVE EXAMPLE 1

A sample of H-ZSM-5 (5.2 g.) having a Si:Al ratio of 56 was loaded into an Incolloy reactor 9 mm ID x 50 cm length. The reactor was equipped with one thermocouple plunged into the catalytic bed. The catalyst was calcined by heating at 450°C for about 3 hours under a nitrogen flow.

The catalyst was then cooled to 290°C and a nitrogen flow of 25 cc/min at atmospheric pressure containing 10% vaporised 1,2-dichloroethane was passed through the catalyst bed with a contact time of 3.6 seconds. The products of the reaction issuing from the reactor were sampled and analyzed using a gas chromatograph equipped with a flame ionization detector. The reaction conditions stabilized in about 1 hour, after which results indicated an EDC conversion of 98-99% with very low selectivity to VCM. After 5 hours the EDC conversion was reduced to 20%.

### COMPARATIVE EXAMPLE 2

The reaction of Example 1 was repeated using as catalyst a sample of Cu-H-ZSM-5 (5.2 g., Cu = 1. 5% w) prepared by an ion exchange method. The same results as Example 1 were obtained both after 1 hour and after 5 hours.

### EXAMPLE 3

The reaction of Example 1 was repeated using as catalyst a sample of Zn-H-ZSM-5 (5.2 g., Zn = 1.4% w), prepared by an ion exchange method, to obtain, after 5 hours, an EDC conversion of 95% with selectivity to VCM of about 97-98%.

### EXAMPLE 4

The reaction of Example 1 was repeated using as catalyst a sample of Mg-H-ZSM-5 (5.2 g., Mg = 0.48% w), prepared by an ion exchange method, to obtain, after 5 hours, an EDC conversion of 95% with selectivity to VCM of about 97-98%.

### EXAMPLE 5

The reaction conditions of Example 3 were modified by using 4.0 g. of the Zn-H-ZSM-5 (Zn = 1.4% w) and a contact time of 1.2 seconds. After 5 hours the EDC conversion was the same as Example 3 with increased selectivity to VCM.

### EXAMPLE 6

The reaction conditions of Example 5 were modified by using a contact time of 0.6 seconds. After 5 hours the EDC conversion was approximately 90% with 99% selectivity to VCM.

### EXAMPLE 7

The reaction conditions of Example 5 were modified by using a contact time of 0.3 seconds to obtain an approximate EDC conversion of 65% with 99.3% selectivity to VCM.

### EXAMPLE 8

The reaction conditions of Example 4 were modified by using 5.2 g. of the Mg-H-ZSM-5 (Mg = 0.48% w) and a contact time of 1.2 seconds. After 5 hours the EDC conversion was about 75% with 98.5% selectivity to VCM.

### EXAMPLE 9

The reaction of Example 4 was repeated using as a catalyst a sample of Mg-H-ZSM-5 (5.2 g.) with a lower Mg²⁺ content (0.24% w instead of 0.48%), always prepared by ion exchange. After 5 hours the EDC conversion was about 75% with 95% selectivity to VCM in comparison with 95% conversion obtained in Example 4.

### EXAMPLE 10

The reaction of Example 3 was repeated working at 260°C. The experiment was repeated for four cycles, spaced by three catalyst reactivation cycles. Approximately the same results were observed in each experimental cycle. Reactivation was performed by heating the catalyst at 450°C for about three hours in an air flow.

### EXAMPLE 11

The reaction of Example 3 was repeated using EDC obtained from an industrial plant (99% purity) without further purification instead of a spectroscopic grade reagent (99.9% purity). The same results as Example 3 were obtained.

### EXAMPLE 12

The reaction of Example 3 was repeated by using a Zn-H-ZSM-5 catalyst (Zn = 1.4% w) prepared by salt impregnation instead of ion exchange. The same results as Example 3 were obtained.

### COMPARATIVE EXAMPLE 13

The reaction conditions of Example 1 were modified by using a S-115 catalyst and working at 325°C. An EDC conversion of about 55% with selectivity to VCM of about 92% was obtained.

### COMPARATIVE EXAMPLE 14

The reaction conditions of Example 13 were modified by working at 290°C to obtain an EDC conversion of about 25% with 95% selectivity to VCM.

### EXAMPLE 15

The reaction conditions of Example 14 were modified by using a Zn-S-115 catalyst to obtain an EDC conversion of about 35% with 99% selectivity to VCM.

## Claims

1. A process for the catalytic dehydrochlorination of ethylene dichloride (EDC) to yield vinyl chloride (VCM) comprising:
(i) a cracking step wherein EDC is brought into contact with a catalyst which is a zeolite having a Si:Al ratio of 10 or more and containing an alkaline earth metal ion or a zinc ion; and
(ii) a collecting step in which VCM is obtained in the reaction products.

2. A process according to claim 1 wherein the cracking step takes place at between 200°C and 400°C.

3. A process according to claim 2, wherein the cracking step takes place at between 250°C and 350°C.

4. A process according to any one of claims 1 to 3 wherein the cracking step takes place at a pressure of between 101.325kPa and 2026.5kPa (1 and 20 atmospheres).

5. A process according to any one of claims 1 to 4 wherein the cracking step comprises a contact time between the zeolite and the EDC of between 0.1 and 5.0 seconds.

6. A process according to any preceding claim wherein the zeolite has a Si:Al ratio of between 10 and 1000.

7. A process according to claim 6 in which the zeolite is ZSM-5 or S-115.

8. A process according to claim 6 or 7 wherein the alkaline earth metal ion is added to the zeolite by an ion exchange process or by impregnation.

9. The use of a zeolite catalyst having a Si:Al ratio of 10 or more and containing a magnesium ion in a process for the catalytic dehydrochlorination of ethylene dichloride to vinyl chloride monomer.

## Patentansprüche

1. Ein Verfahren zur katalytischen Dehydrochlorierung von Ethylendichlorid (EDC) zur Gewinnung von Vinylchlorid (VCM), welches Verfahren umfaßt:
(i) eine Krackstufe, in welcher EDC mit einem Katalysator in Kontakt gebracht wird, der aus einem Zeolith besteht, der ein Si:Al-Verhältnis von 10 oder höher aufweist und ein Erdalkalimetallion oder ein Zinkion enthält; und
(ii) eine Auffangstufe, in welcher VCM in den Reaktionsprodukten erhalten wird.

2. Ein Verfahren nach Anspruch 1, in welchem die Krackstufe zwischen 200°C und 400°C stattfindet.

3. Ein Verfahren nach Anspruch 2, in welchem die Krackstufe zwischen 250°C und 350°C stattfindet.

4. Ein Verfahren nach einen, der Ansprüche 1 bis 3, in welchem die Krackstufe bei einem Druck zwischen 101,325 kPa und 2026,5 kPa (1 und 20 Atmosphären) stattfindet.

5. Ein Verfahren nach einem der Ansprüche 1 bis 4, in welchem die Krackstufe eine Kontaktzeit zwischen dem Zeolith und dem EDC zwischen 0,1 sec und 5,0 sec umfaßt.

6. Ein Verfahren nach irgendeinem vorhergehenden Anspruch, in welchem der Zeolith ein Si:Al-Verhältnis zwischen 10 und 1000 hat.

7. Ein Verfahren nach Anspruch 6, in welchem der Zeolith ZSM-5 oder S-115 ist.

8. Ein Verfahren nach Anspruch 6 oder 7, in welchem das Erdalkalimetallion zu dem Zeolith durch ein Ionentauscherverfahren oder durch Imprägnierung zugesetzt ist.

9. Die Verwendung eines Zeolith-Katalysators, der ein Si:Al-Verhältnis von 10 oder höher aufweist und ein Magnesiumion enthält, in einem Verfahren zur katalytischen Dehydrochlorierung von Ethylendichlorid zu Vinylchlorid-Monomer.

## Revendications

1. Procédé pour la déshydrochloration catalytique du dichlorure d'éthylène (EDC) pour donner dit chlorure de vinyle (VCM), comprenant :
(i) une étape de craquage dans laquelle de l'EDC est mis en contact avec un catalyseur qui est une zéolite ayant un rapport Si:Al de 10 ou plus et contenant un ion de métal alcalino-terreux ou un ion zinc ; et
(ii) une étape de collecte dans laquelle du VCM est obtenu dans les produits de réaction.

2. Procédé selon la revendication 1, dans lequel l'étape de craquage se déroule entre 200 °C et 400 °C.

3. Procédé selon la revendication 2, dans lequel l'étape de craquage se déroule entre 250 °C et 350 °C.

4. Procédé selon l'une quelconque des revendications 1 à 3, dans lequel l'étape de craquage se déroule à une pression entre 101,325 kPa et 2026,5 kPa (1 et 20 atmosphères).

5. Procédé selon l'une quelconque des revendications 1 à 4, dans lequel l'étape de craquage comprend un temps de contact entre la zéolite et l'EDC compris entre 0,1 et 5,0 secondes.

6. Procédé selon l'une quelconque des revendications précédentes, dans lequel la zéolite a un rapport Si:Al entre 10 et 1000.

7. Procédé selon la revendication 6, dans lequel la zéolite est la ZSM-5 ou la S-115.

8. Procédé selon la revendication 6 ou 7, dans lequel l'ion de métal alcalino-terreux est ajouté à la zéolite par un procédé d'échange d'ions ou par imprégnation.

9. Utilisation d'un catalyseur zéolitique ayant un rapport Si:Al de 10 ou plus et contenant un ion magnésium, dans un procédé pour la déshydrochloration catalytique du dichlorure d'éthylène en monomère chlorure de vinyle.
